# EUROPEAN PATENT APPLICATION

(11) **EP 2 123 314 A1**
(43) Date of publication of application: **25.11.2009**
(21) Application number: 07800879.4
(22) Date of filing: 06.09.2007
(51) Int. Cl.: A61M 1/14, B01D 61/00, C02F 1/44

(54) **CONTINUOUSLY WORKING AND PORTABLE HAEMODIALYSIS APPARATUS**

(30) Priority: 28.02.2007 CN 200710000488
(71) Applicant: Alkanhal, Fahad Ahmed, Beijing 100013 (CN)
(72) Inventor: Alkanhal, Fahad Ahmed, Beijing 100013 (CN)
(74) Representative: Warrilow, David Thomas
(86) International application number: PCT/CN2007/002663
(87) International publication number: WO 2008/104108

(57) **Abstract**

This invention discloses a Continuous Ambulatory Haemofiltration (CAHF) Device, consisting of an outer casing, a multifunction pump, a haemofilter, a reversed osmosis filter, a power source, a drainage bag, blood lines and fluid lines, characterizes in that the haemofiltrate from the haemolfilter is moved to a reversed osmosis filter. The ultrafiltrate fluid from the reversed osmosis filter flows to the out-flow tube of the haemofilter veineus line through a specific fluid line. The exit of the reversed osmosis filter is connected to a drainage bag. The role of the a multifunction pump is to move the blood from the jagular perm catherter to the haemofilter, and to move fluids between the haemofilter, reversed osmosis filter, veineus line and the drainage bag. An electrode is placed at the in-flow tube of the haemofilter to measure the incoming blood osmolality. The electrode is connected to a microprocessor that is further connected to a computer-controlled valve that is placed at the out-flow line of the reversed osmosis filter. The microprocessor is connected with a memory card. The device is much smaller than a conventional device, and can produce haemofiltration replacement solution itself. Using the device can decrease the agony and medical care cost of a patient to a great extent.

## Description

### Technical field

This invention relates to a medical apparatus, particularly a Continuous Ambulatory Haemofiltration Device.

### Technical background

The kidney has many vital functions, which include removal of excessive water and electrolytes and removal of metabolic waste product. When kidney fails to do their functions, a patient is going to die unless he receives renal replacement therapy.

The available renal replacement therapy these days are the following:
1. Renal transplantation but is limited by shortage of graft kidneys, in addition patients have to be put on expensive immunosuppressive medications that have a lot of side effects and need to be regularly monitored and there are many contraindication of renal transplantation.
2. Peritoneal dialysis which requires large amount of expensive fluids and other disposables. In addition it has many complications and efficiency of the peritoneal membrane decreases markedly with the time and this type of renal replacement therapy requires daily effort from the patient for fluid exchange.
3. So large numbers of end stage renal failure patients accumulate to receive conventional haemodialysis which requires large number of nursing staff, a lot of expensive fluids and disposables. In addition patients need to come to haemodialysis unit about 3 times per week and need to be pricked by 2 large needles each haemodialysis session plus that this type of therapy has a lot of complications. In haemodialysis units conventional haemofiltration and haemodiafiltration can be performed but they need even more expensive fluids and disposables.

The present invention decreases the medic care cost, inconvenience and agony of renal failure patients.

### Description of the invention

The object of the present invention is to provide a small-sized, ambulatory haemofiltration device that can produce hameofiltration replacement solution itself.

The technical scheme to achieve the invention is as follows:

A Continuous Ambulatory Haemofiltration (CAHF) Device, consisting of an outer casing, a multifunction pump, a haemofilter, a reversed osmosis filter, a power source, a drainage bag, blood lines and fluid lines, characterizes in that blood moves from a patient to haemofilter, then the the haemofiltrate produced in will be moved to a reversed osmosis filter. The ultrafiltrate fluid from the reversed osmosis filter flows to the out-flow tube of the haemofilter veineus line through a specific fluid line. The exit of the reversed osmosis filter is connected to a drainage bag. The role of the a multifunction pump is to move the blood from the jagular perm catherter to the haemofilter, and to move fluids between the haemofilter, reversed osmosis filter, veineus line and the drainage bag. An electrode is placed at the in-flow tube of the haemofilter to measure the incoming blood osmolality. The electrode is connected to a microprocessor that is further connected to a computer-controlled valve that is placed at the out-flow line of the reversed osmosis filter. The microprocessor is connected with a memory card.

The said in-flow line to and out-flow line of and ultrafiltrate line from the reversed osmosis filter are connected to a volumetric device to measure the volumes of fluids going through those lines. The volumetric device is connected with the microprocessor that is connected to the memory card. The said power source is in the form of 2 rechargable batteries that are connected in parallel form.

The said multifunctional pump is a coaxial (axle-sharing) multifunctional pump. There are two impellers driven by one micro electric motor. The one impeller impels the blood from the patient's body to the haemofilter, and the haemofiltrate from the the haemofilter to the reversed osmosis filter. The other impeller impels the ultrafiltrate (product fluid) from the reversed osmosis filter to the tube connected with the patient's body vein. The haemofilter and the reversed osmosis filter are placed in tunnels at either side of the casing, and other parts are placed in the centre between the two tunnels.

There are 2 power indicating alarms on the outer casing of the device. One indicates low blood flow through the haemofilter and the other one indicates low battery power.

There is a binding belt on the said outer casing.

A valve is placed at the fluid line that connecting the reversed osmosis filter and the inlet of haemofilter.

A valve is placed at the fluid line that connecting the reversed osmosis filter and haemofilter itself. **The branch of the fluid line carrying the dialysate to the hameofilter itself must enter to the haemofilter through the upper end of one side of the haemofilter to the dialysate compartment.**

A store of mixed powder of sodium chloride and sodium biacarbonate is provided to add enough sodium chloride or biacarbonate to the ultrafiltrate from the reversed osmosis filter.

The present device with a simple and portable structure can be easily bound to a patient' s body. When the power-indicating alarm is activated, one of the battery can be replaced without affecting the continuous operation of the device as the two batteries are conntected in parallel form. If blood flow alarm light on, it indicates a failure of the device function and patient must go to the hospital immediately. The haemofiltrate from the haemofilter is moved to a reversed osmosis filter. The ultrafiltrate from reversed osmosis filter flows back to a patient' s body as replacement solution. This greatly reduces the medic care cost of a patient.

### Figures:

Figure 1 is a structural figure of the inside of the device;
Figure 2 is an illustrative figure of the back of the device;

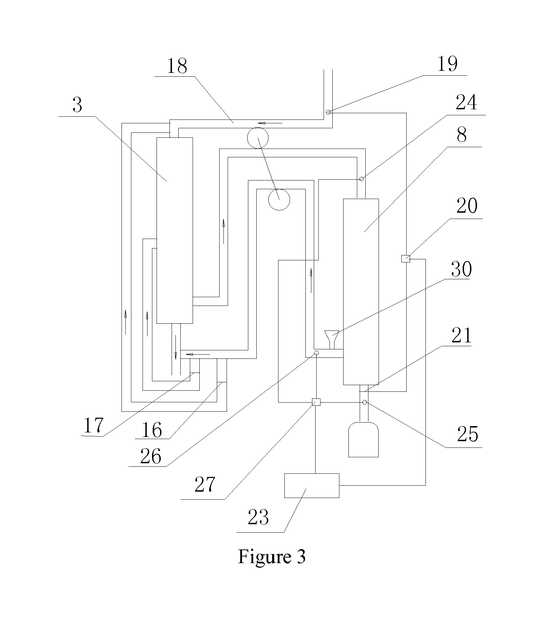

Figure 3 is an illustrative figure of the connection of major parts of the device;

### Preferred embodiment

The present invention is better understood illustrated with the figures:

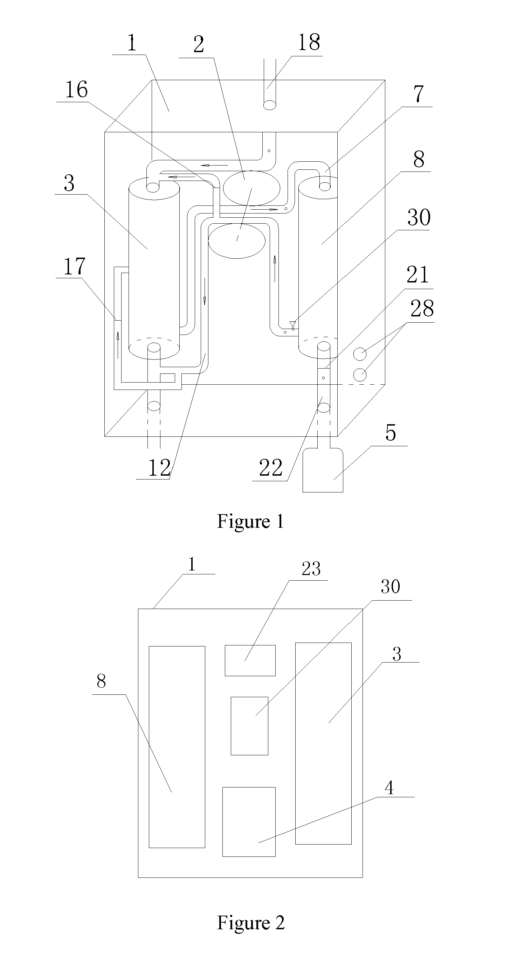

As shown in Figures 1 and 2, the Continuous Ambulatory Haemofiltration (CAHF) Device consists of an outer casing(1), a multifunction pump(2), a haemofilter(3), a power source(4), a drainage bag(5), blood lines and fluid lines. The haemofiltrate from the haemolfilter(3) is moved to a reversed osmosis filter(8) through fluid line(7). The exit of the reversed osmosis filter(8) is connected to a drainage bag(5). The ultrafiltrate from the reversed osmosis filter(8) is connected to the inlet, exit of haemofilter(3) and haemofilter(3) itself via fluid line(12) respectively. Valves (16 and 17) are placed at the fluid lines that connecting the reversed osmosis filter(8) and the inlet of haemofilter(3) and haemofilter(3) itself respectively. **The branch of the fluid line carrying the dialysate to the hameofilter (3) itself must enter to the haemofilter(3) through the upper end of one side of the haemofilter(3) to the dialysate compartment.** The multifunction pump (2) moves the blood from the jagular perm catherter(18) to the haemofilter(3), and to move fluids between the haemofilter(3), reversed osmosis filter(8), fluid line(12) and the drainage bag(5). The multifunction pump(2) has two impellers driven by one micro electric motor. One impeller impels the blood from the patient's body to the haemofilter(3), and the haemofiltrate from the the haemofilter(3) to the reversed osmosis filter(8). The other impeller impels the ultrafiltrate (product fluid) from the reversed osmosis filter(8) to the tube connected with fluid line(12). An electrode(19) is placed at the in-flow tube of the haemofilter(3) to measure the incoming blood osmolality. The electrode(19) is connected to a microprocessor(20) that is further connected to a computer-controlled valve(21) that is placed at the out-flow line of the reversed osmosis filter(8). The microprocessor is connected with a memory card(23). Electrodes (24, 25 and 26) are placed at the inlet, exit and out-flow line of ultrafiltrate of the reversed osmosis filter(8) respectively. The said 3 electrodes are connected to volumetric devices to measure fluids going through those lines. The volumetric devices are connected with a microprocessor(27) to send those information to memory card(23). The said power source(4) is composed of 2 rechargeable batteries connected in parallel form. The haemofilter(3) and the reversed osmosis filter(8) are placed in tunnels at either side of the casing, and other parts are placed in the centre between the two tunnels. Two light alarms(28) are placed on the outer casing(1). One indicates low blood flow through haemofilter, and the other one indicates low battery power. A binding belt(29) is mounted on the outer casing(1). A store of mixed powder of sodium chloride and sodium biacarbonate (30) is provided to add enough sodium chloride or biacarbonate to the ultrafiltrate from the reversed osmosis filter(8).

In order to make this invention better understood, the working process of the device is concisely described hereunder. Blood flows from the jagular perm catherter to the haemofilter. The purified blood flows back to the patient's body. The haemofiltrate is moved to the reversed osmosis filter. The rejected fluid by the reversed osmosis filter flows to the drainage bag, while the ultrafiltrate flows back to the patient's body as replacement solution. To avoid clotting in the haemofilter, part of the ultrafiltrate (20-30%) from the reversed osmosis filter must be connected to incoming blood to the haemofilter. Part of the ultrafiltrate can also be connected to the haemofilter itself as dialysate. The percentage of ultrafiltrate to the inlet of haemofilter or to the haemofilter itself can be adjusted by a doctor by adjusting the valves on the lines going to these sites. An electrode is placed at the in-flow tube of the haemofilter to measure the incoming blood osmolality, and feedbacks the measurement to a microprocessor that controls the operation of a valve being placed at the out-flow line of the reversed osmosis filter, and thereby controls the volume of fluid flowing into the patient's body according to patient's blood osmolality. All relevant information is stored in the memory card. Electrodes (24, 25 and 26) are placed at the inlet, exit and out-flow line of ultrafiltrate of the reversed osmosis filter respectively. The said 3 electrodes are connected with volumetric device and with a microprocessor(27) that stores information regarding the amount of fluids going in and out of the reversed osmosis filter in memory card(23). In this way, a patient needs not to see a doctor frequently, and he/she only needs to see a doctor once a month to have the development monitored by a doctor who checks the information in the memory card using a computer. If needed, a store of mixed powder of sodium chloride and sodium biacarbonate (30) is provided to add enough sodium chloride or biacarbonate to the ultrafiltrate from the reversed osmosis filter. The device is fixed to the right side of the chest of the patient, or other adequate place, by a binding belt where it can be connected to the right-sided jagular perm catheter.

## Claims

1. A Continuous Ambulatory Haemofiltration (CAHF) Device, consisting of an outer casing, a multifunction pump, a haemofilter, a reversed osmosis filter, a power source, a drainage bag, blood lines and fluid lines, **characterizes in that** the haemofiltrate from the haemolfilter is moved to a reversed osmosis filter. The ultrafiltrate fluid from the reversed osmosis filter flows to the out-flow tube of the haemofilter veineus line through a specific fluid line. The exit of the reversed osmosis filter is connected to a drainage bag. The role of the a multifunction pump is to move the blood from the jagular perm catherter to the haemofilter, and to move fluids between the haemofilter, reversed osmosis filter, veineus line and the drainage bag. An electrode is placed at the in-flow tube of the haemofilter to measure the incoming blood osmolality. The electrode is connected to a microprocessor that is further connected to a computer-controlled valve that is placed at the out-flow line of the reversed osmosis filter. The microprocessor is connected with a memory card.

2. A Continuous Ambulatory Haemofiltration Device according to claim 1, **characterizes in that** the in-flow line to and out-flow line of and ultrafiltrate line from the reversed osmosis filter are connected to a volumetric device to measure the volumes of fluids going through those lines. The volumetric device is connected with the microprocessor that is connected to the memory card.

3. A Continuous Ambulatory Haemofiltration Device according to claim 1 and 2, **characterizes in that** the said power source is in the form of 2 rechargable batteries that are connected in parallel form.

4. A Continuous Ambulatory Haemofiltration Device according to claim 1 and 2, **characterizes in that** the said multifunctional pump is a coaxial (axle-sharing) multifunctional pump. There are two impellers driven by one micro electric motor. The upper (or the lower) impeller impels the blood from the patient's body to the haemofilter, and the haemofiltrate from the the haemofilter to the reversed osmosis filter. The other impeller impels the ultrafiltrate (product fluid) from the reversed osmosis filter to the tube connected with the patient's body vein.

5. A Continuous Ambulatory Haemofiltration Device according to claim 1, **characterizes in that** the haemofilter and the reversed osmosis filter are placed in tunnels at either side of the casing, and other parts are placed in the centre between the two tunnels.

6. A Continuous Ambulatory Haemofiltration Device according to claim 1, **characterizes in that** the device is fixed to the right side of the chest of the patient by a binding belt where it can be connected to the right-sided jagular perm catheter.

7. A Continuous Ambulatory Haemofiltration Device according to claim 1, **characterizes in that** part of the ultrafiltrate (20-30%) from the reversed osmosis filter can be connected to incoming blood to the haemofilter to dilute the blood to avoid clotting in the haemofilter.

8. A Continuous Ambulatory Haemofiltration Device according to claim 1, **characterizes in that** ultrafiltrate from the reversed osmosis filter can be used as dialysate to perform haemodialysis or haemodiafiltration if needed. The branch of the fluid line carrying the dialysate to the hameofilter itself must enter to the haemofilter through the upper end of one side of the haemofilter to the dialysate compartment.

9. A Continuous Ambulatory Haemofiltration Device according to claim 1, **characterizes in that** a store of mixed powder of sodium chloride and sodium biacarbonate can be provided to add enough sodium chloride or biacarbonate to the ultrafiltrate from the reversed osmosis filter if needed.

10. A Continuous Ambulatory Haemofiltration Device according to claim 1, **characterizes in that** there are 2 light alarms on the outer casing of the device. One indicates the low blood flow in the device, and the other one indicates low battery power.
